# EUROPEAN PATENT APPLICATION

(11) **EP 4 360 640 A1**
(43) Date of publication of application: **01.05.2024**
(21) Application number: 23205177.1
(22) Date of filing: 23.10.2023
(51) Int. Cl.: A61K 36/32, A61K 38/48, A61K 31/185, A61K 31/704, A61K 36/53, A61P 13/00, A61P 13/04, A61P 13/12

(54) **COMPOSITION FOR THE TREATMENT AND PREVENTION OF URINARY TRACT STONES**

(30) Priority: 24.10.2022 IT 202200021921
(71) Applicant: Anvest Group S.r.l., 20152 Milano (IT)
(72) Inventor: ALFIERI, Sandro, 84083 Castel San Giorgio (IT); ALFIERI, Dario, 84086 Roccapiemonte (IT); ALFIERI, Roberto, 84086 Roccapiemonte (IT)
(74) Representative: Cattaneo, Elisabetta

(57) **Abstract**

The invention relates to a composition comprising potassium citrate, magnesium citrate, a phyllanthus extract, bromelain, a nettle extract, a rustyback extract, an origanum extract and a Protium heptaphyllum extract. The composition of the invention, in the form of a medicament or dietary supplement, is useful in the treatment and prevention of urinary tract stones.

## Description

### FIELD OF THE INVENTION

The present invention relates to a composition comprising extracts of natural ingredients for the treatment and prevention of urinary tract stones.

### BACKGROUND OF THE INVENTION

Urinary tract stones, also referred to as nephrolithiasis or urolithiasis, are a pathological condition in which there are stones of varying size and composition in the urinary tracts (bladder, urethra, kidneys, etc.). Urolithiasis is the third most common urological disease after urinary tract infections and prostate diseases, and has an occurrence rate of about 15%, which is continuing to increase.

The increase in the incidence of kidney stones observed in recent decades is most likely caused by changes in eating habits, particularly in relation to a high-protein, high-sodium and high-sugar diet, and lifestyle in all industrialized countries where physical activity has generally been reduced. Moreover, urinary tract stones occur more frequently, and are therefore diagnosed more in men than in women.

Stone formation occurs through supersaturation of minerals in the urine, which precipitate and form crystals in the renal calyces and subsequent urinary tracts. The precipitation of supersaturated solutes is a well-known concept in common practice, which also applies in the human body in the case of supersaturation of minerals in urine, which should be eliminated through excretion.

Conditions that may lead to stone formation are inflammation, oxidant-antioxidant imbalance, angiogenesis, purine metabolism and urea cycle disorders. They play a crucial role in the development of nephrolithiasis. Urine mainly contains citrates in order to facilitate the dissolution of salts, without the risk of them precipitating. However, under certain conditions, this balance is compromised, resulting in the formation of stones.

The composition of the stones can vary considerably depending on several factors:
- The vast majority (around 90%) of stones in the western world are calcium oxalate stones, which are radiopaque, i.e. visible on X-rays.
- Another, less frequent type is that of calcium phosphates, also visible on X-rays.
- Uric acid stones are increasingly common and are not visible on standard X-rays. They are recognisable by ultrasound or CT scan and their composition makes them particularly susceptible to dissolution by urine alkalinisation therapy.
- Cystine stones are rare and often occur at a very young age when infants present with a condition called cystinuria. Cystine precipitates are difficult to treat and require complex multidisciplinary therapies.

Stones can also be distinguished according to the presence of a calcic or non-calcic phase. Stones in the calcic phase include stones composed of calcium oxalates and phosphates (the presence of calcium gives the term its name). Non-calcium stones essentially consist of uric acid stones or infected or cystine stones.

When the size of the stones is such that they block the renal tract or ureter, renal colic occurs, which means episodes of severe pain in the side that may also extend to the abdomen, sometimes accompanied by other secondary symptoms such as difficulty urinating, fever and vomiting. This acute symptomatology is linked not so much to the presence of the stone in the urinary tracts, but to the urinary system's attempt to eliminate it. In fact, a small kidney stone that does not move, does not obstruct and is not infected generally does not lead to considerable symptoms or generates moderate pain. On the other hand, when the stone moves into the ureter, the ureter contracts to facilitate its descent. Repeated contractions in order to expel the stone obstructing the ureter generate the pain. Renal colic is the typical manifestation of ureteral stones. The pain can be so violent that it can be compared to childbirth pain. It begins in its typical form in the lumbar region and radiates anteriorly downwards towards the inguinal region and can also affect the scrotum in males. Colic pain is a cramp-like pain with an intermittent pattern: it gradually increases to a peak and then slowly subsides. The patient presents very agitated, sometimes with fever due to urinary stasis, vomiting and blood in urine. On ultrasound, the stone and/or a dilated urinary tract can be seen. As mentioned above, X-rays of the abdomen always show the stone, unless it consists of uric acid.

The formation of calcium oxalate and calcium phosphate kidney stones is the result of cellular and extracellular events. Extracellular events comprise:
- supersaturation (mineral precipitation)
- nucleation (crystal aggregation)
- growth (layering of concentric levels)
   The crystals pass through these three stages in the renal tubular lumen and renal pelvis. Cellular events occur in the cells of the renal epithelium and interstitium and comprise:
   - management of acid-base balance, urinary citrate, oxalate, calcium, magnesium and pH.
   - the response of renal epithelial cells to changes in the urinary environment.

Since the early 1950s, research aimed at solving the problem of kidney stones has always focused on the composition of the minerals themselves and how their formation leads to disease progression.

There is therefore a need to broaden our knowledge of stones by researching what cellular mechanisms take place on the urinary epithelium and kidney cells when there is an accumulation of insoluble mineral precipitate. The interaction between renal epithelial cells and calcium phosphate and/or calcium oxalate crystals in turn alters renal cell functions, changes the extracellular environment and plays a significant role in the formation of calcium oxalate stones. Exposure to high levels of oxalate crystals and calcium phosphates is harmful to kidney cells. In the long run, this condition can lead to cell death, which causes the formation of membranous vesicles that leave the epithelium and end up in the urine. After this phase of desquamation of the epithelium, the crystals are internalized within the cell membrane to which they are anchored. Under normal conditions, the cells are resistant to the attachment of oxalate crystals. Disruption of the tight junctions is the main cause of this alteration and exposes crystals to anchoring, which can lead to stones. Some molecules involved in crystal binding to the urinary epithelium and kidney cells are hyaluronic acid and osteopontin.

In most cases, the prevention of stones consists of reducing the incidence of recurrence after previous treatment to remove precipitates in the urinary tract. Generally speaking, the best strategy for preventing kidney stones is a significant daily intake of fluids, especially low-mineral waters. Some sources recommend drinking 2-3 litres of water a day, but this practice should only be strictly followed in cases where there is a clear predisposition of the subject to relapse. The intake of citrates is a valid treatment for reducing the precipitation of solutes in the urinary tract, as well as the intake of other extracts that have demonstrated oxalate and phosphate breakdown capabilities.

In the case of severe pain caused by stones in the acute phase, intravenous antispasmodics, painkillers and anti-inflammatory drugs are administered awaiting the spontaneous expulsion of the stone from the ureter into the bladder. In most cases, once in the bladder, the stone easily reaches the urethra without obstructing it since it is larger than the ureter. To facilitate the spontaneous expulsion of the stone, it is recommended to drink 1 litre of water in 15 minutes (water shot), in this way the buoyancy of the liquids coming out of the bladder promotes the expulsion of ureteral sediments.

In more complex cases of infected or blocked stones in the ureter, the most common interventions are:
- Endoscopic removal: an optical probe inserted along the ureter locates the stone, which is crushed with a laser beam or ultrasound.
- Extracorporeal lithotripsy consists of bombarding the stone with shock waves generated by a lithotripter outside the body. This type of operation is used for small stones, since once crushed, they must be removed naturally through the urethra.
- Percutaneous renal lithotripsy is used in cases where the first two routes are not possible. It is performed under general anaesthesia through a hole in the hip that allows direct access to the stone.
- Laparoscopic operation, less invasive than open surgery, aimed at removing the stone by direct access.
- Open surgery consists of opening the abdomen operated in 5% of cases for stones that cannot be crushed or of considerable size, such as those spread throughout the pelvis or the renal pelvis.

Clearly, there is a need not only to treat formed stones, but also to prevent their formation.

Hypocitraturia or low urinary excretion of citrate is a common feature in patients with nephrolithiasis, particularly those with calcium stone disease (calcium oxalates and phosphates). Citrate is a weak acid that is synthesized within the Krebs cycle. It can also enter the body through dietary intake. Various metabolic abnormalities, such as metabolic acidosis, hypokalaemia and starvation, appear to influence the renal utilisation of citrate by inducing a decrease in urinary excretion of citrate. Hypocitraturia is defined as urinary citrate excretion of less than 320 mg/day. Citrate plays a key role in reducing stone formation as it reduces urinary supersaturation of calcium salts. In particular, calcium (and magnesium) citrate complex is much more soluble in urine than calcium oxalate and calcium phosphate, which significantly reduces the formation of crystals that can clog the urinary tracts.

The aim of the present invention is therefore to treat urinary tract stones effectively by overcoming the adverse effects of the prior art.

### SUMMARY OF THE INVENTION

The inventors have found a combination of active substances that was able to effectively treat stones. In particular, the inventors have identified a combination of substances, some already known for the treatment of stones, but which when combined produce a greater synergistic effect than the additive combination of known substances as will become evident from the experimental part.

The invention thus relates to a composition comprising potassium citrate, magnesium citrate, a phyllanthus extract, bromelain, a nettle extract, a rustyback extract, an origanum extract and a Protium heptaphyllum extract.

The invention also relates to a composition comprising potassium citrate, magnesium citrate, a phyllanthus extract, bromelain, a nettle extract, a rustyback extract, an origanum extract and a Protium heptaphyllum extract for use as a medicament. According to a further aspect, the invention relates to a composition comprising potassium citrate, magnesium citrate, a phyllanthus extract, bromelain, a nettle extract, a rustyback extract, an origanum extract and a Protium heptaphyllum extract for use in the treatment and prevention of stones.

The invention thus also concerns a supplement comprising the composition of the invention and excipients for use in the treatment of stones.

Advantageous aspects of the composition and of the supplement of the invention, such as amount, posology and dosages will be indicated in detail in the detailed description and the surprising synergistic effects deriving therefrom will become evident from the next experimental part.

In an advantageous aspect of the invention the composition of the invention may be combined in the treatment of stones with at least one drug chosen from the group consisting of allopurinol, a diuretic, an antispasmodic, an antibiotic and a non-steroidal anti-inflammatory drug (NSAID).

The composition of the invention may in fact be administered alone or in combination, i.e. as a co-therapeutic agent in a fixed-dose combination or in a combined therapy of separately formulated active ingredients, with at least one drug, such as, for example, allopurinol, a diuretic, an antispasmodic, an antibiotic and a non-steroidal anti-inflammatory drug (NSAID) for the resolution of symptoms resulting from urinary tract stones.

The composition, or the supplement comprising the same, of the invention alone or advantageously in combination with at least one drug selected from the group consisting of allopurinol, a diuretic, an antispasmodic, an antibiotic and a non-steroidal anti-inflammatory drug (NSAID) was found to be effective in the treatment of stones, in particular with respect to the symptoms associated with it.

In fact, the composition of the invention, or the medicament or supplement comprising the same, exhibits excellent activity in the prevention and treatment of kidney stone formation, myorelaxation, regulation of uric acid precipitation, oxalate crystal scaling and anti-edema effect. Specifically, the product of the invention exhibited excellent inorganic breakdown activities of already precipitated oxalates, a characteristic that suggests its effective use in the treatment of acute stones. In addition, the invention exhibited excellent activity in reducing the precipitation of calcium oxalates in a supersaturated environment, which suggests its effective use in the prevention of stones.

### DESCRIPTION OF THE DRAWINGS

Figure 1 shows the increased vascular permeability and subsequent edema after ischemic damage induced by bilateral renal artery occlusion for 30 minutes and subsequent reperfusion, resulting in fluorescent dextran extravasation and vasoconstriction. Specifically, Figure 1 shows the arterial circulation and edema effect before (a) and after occlusion and ischemic damage (b).
Figure 2 shows the arterial circulation and edema effect before (a) and after occlusion and ischemic damage (b), following the administration of bromelain.
Figure 3 shows the arterial circulation and edema effect before (a) and after occlusion and ischemic damage (b), following administration of the composition of the invention.
Figure 4 shows the intracellular fluorescence of Ca²⁺ ([Ca²⁺]ᵢ) in cultured smooth muscle cells (SMCs). SMCs were grown in confocal plates and incubated with Fluo-3/am (5 µM) at room temperature. After Fluo-3/am was metabolized in the cells for 20 min, the SMCs were stimulated by acetylcholine (ACh, 10-6 mM) for 10 s and then perfused with buffer containing Ca²⁺or buffer free from Ca²⁺, respectively. [Ca²⁺]ᵢ of SMC was scanned continuously by LSM 510 (ZEISS). (a) Control; (b) nettle + oregano; (c) composition of the invention.
Figure 5 shows the effects of active ingredients on the expression of URAT1 and OAT1 in the HK2 cell. The cells were treated for 24 hours. The protein expression levels of URAT1 and OAT1 were analysed by western blotting. β-actin was used as a loading control and the results are representative of at least three independent experiments. Each bar represents the mean ± SD (n=3). *P < 0.05, **P < 0.01 with respect to the control group.
Figure 6 shows the light microscopy of calcium oxalate crystals, induced in synthetic human urine by adding sodium oxalate, in the absence (Ctrl) and in the presence of Phyllanthus and the whole mixture (400 X magnification).
Figure 7 shows the number of calcium oxalate crystals analysed by automatic counting, classified by size, in the absence and presence of active ingredients in synthetic human urine.

### DETAILED DESCRIPTION OF THE INVENTION

The invention relates to a composition comprising potassium citrate, magnesium citrate, a phyllanthus extract, bromelain, a nettle extract, a rustyback extract, an origanum extract and a Protium heptaphyllum extract.

The invention envisages the presence of potassium citrate and magnesium citrate. Hypocitraturia or low urinary excretion of citrate is a common feature in patients with nephrolithiasis, particularly those with calcium stone disease (calcium oxalates and phosphates). Citrate is a weak acid that is synthesized within the Krebs cycle. It can also enter the body through dietary intake. Various metabolic abnormalities, such as metabolic acidosis, hypokalaemia and starvation, appear to influence the renal utilisation of citrate by inducing a decrease in urinary excretion of citrate. The composition of the invention containing the two citrate salts is able to play a key role in reducing stone formation as it reduces urinary supersaturation of calcium salts. In particular, calcium (and magnesium) citrate complex is much more soluble in urine than calcium oxalate and calcium phosphate, which significantly reduces the formation of crystals that can clog the urinary tracts.

Advantageously, the potassium citrate salt is present in the composition of the invention in the range from 1000 to 2500 mg, preferably about 2000 mg with respect to a unit preparation dose.

Advantageously, the magnesium citrate salt is present in the composition of the invention in the range from 500 to 1500 mg, preferably about 1000 mg, with respect to a unit preparation dose.

The composition of the invention comprises a phyllanthus extract, preferably including 15% tannins.

Phyllanthus, a plant belonging to the Euphorbiaceae family, has more than 50 active ingredients identified as alkaloids, flavonoids (especially tannins), lignans and triterpenes. Among these active ingredients, triterpenes play a role in inhibiting calcium oxalate-induced cytotoxicity. In addition, the tannins it contains participate in the breakdown of calcium oxalate and calcium phosphate stones. The composition of the invention due to the presence of phyllanthus allows the three-dimensional arrangement of calcium oxalate crystals to be maintained. This capacity of phyllanthus makes the composition of the invention very useful for the prevention of relapses of stones and as a preventive therapy aimed at reducing the incidence of stones in subjects with a hereditary risk. As analysed above, a small, non-infected stone that does not obstruct the urinary tracts (especially the ureter) does not pose a health risk and does not cause renal colic. This is another reason why drastically reducing the aggregation of oxalate crystals through the action of the composition of the invention in the urinary tracts is a valid therapeutic strategy to minimize the risk of stones.

Phyllanthus extract, preferably comprising 15% tannins, comprises an amount of phyllanthus in the range of 200 to 400 mg, preferably about 300 mg, with respect to a unit preparation dose.

The composition of the invention comprises bromelain.

Bromelain is an enzyme obtained from the juice and stem of the pineapple. As an enzyme, bromelain has a protein structure composed of amino acids assembled together in a three-dimensional structure. Specifically, bromelain is a proteolytic enzyme, i.e. a protein that can "digest" other proteins by cutting the peptide bonds that hold its amino acid chains together. The breaking of the peptide bond occurs via a water molecule intervening between the carboxyl group and the amino group of the amino acids that make up the protein. Due to its protease nature, bromelain is able to effectively exert anti-edema and anti-inflammatory actions. In particular, edema, i.e. localized swelling in a certain area of the body that is generated as a result of trauma (stab wound, puncture wound, chronic inflammation, etc.), is due to the accumulation of fluid that has arrived in those tissues from the blood vessels to carry immune system organelles and inflammatory molecules. The liquids that form the edema are held in place by protein structures composed of fibrin. The composition of the invention comprising bromelain is thus able to break down these protein structures, allowing the edema to be reabsorbed and move away from the district that previously housed the inflammation. In addition, the composition of the invention, thanks to the presence of bromelain, stimulates diuresis, allowing considerable volumes of fluid to be removed through excretion with urine. These characteristics of the composition make it useful in cases of stones, since the infection that often accompanies stone exacerbation causes edema in the urinary tract and stagnation of fluid. In addition, invasive treatments that use lasers or shock waves to crush the stone cause widespread edema in the urinary tract and difficulty in removing fluid.

The composition of the invention comprises bromelain in an amount in the range of 100 to 250 mg, preferably about 200 mg with respect to a unit preparation dose.

The composition also comprises nettle extract.

Uric acid is the final metabolite of the digestion of purines, nitrogenous bases found mainly in certain foods, but also contained in the DNA of cells. Therefore, both through diet and normal cell metabolism, uric acid is produced. For the human body, uric acid is a waste product and therefore, excess uric acid in the blood must be eliminated by the kidneys through urine. However, when it is produced in excessive quantities or if it is not adequately removed, it can accumulate in the blood leading to hyperuricemia. Hyperuricemia is also an independent risk factor for coronary heart disease, hypertension, diabetes and other metabolic diseases. Uric acid stones account for about 5-10% of kidney stones and are often formed in individuals who have very acidic urine, resulting in the precipitation of uric acid into crystals. The transporters involved in the regulation of uric acid in the proximal tubule of the kidney, together with other proteins present on both the basolateral membrane and the apical membrane of the proximal renal tubule, are involved in the transport of the substance to regulate its level in the plasma. URAT1 is the main mediator of uric acid transport from the kidney to the blood. Inhibition of URAT1 reduces uric acid levels in the blood due to its passage to the renal tubule for elimination via the urine. Conversely, the transporters OAT1 and OAT3 are primarily responsible for the passage of uric acid from the blood to the renal tubule. Thus, by stimulating their functioning, the reduction of hyper-uricemia is promoted. Thanks also to the presence of nettle extract, the composition of the invention has a regulatory action on these processes, significantly reducing the activity of the enzyme xanthine oxidase (XOD); it also inhibits the expression of URAT1 and thus increases the passage of uric acid in the urine to promote its removal in a basic environment without causing its precipitation. At the same time, the composition containing nettle extract also stimulates the action of OAT1, maximizing the removal of uric acid via the urine.

The composition of the invention comprises nettle extract in an amount in the range of 25 to 100 mg, preferably about 50 mg, with respect to a unit preparation dose.

The composition also comprises a rustyback extract.

*Ceterach officinarum,* known as rustyback, is a plant that is widespread throughout Europe. The composition of the invention comprising said extract exhibits antilithic activity, i.e., an action of inhibiting the formation of calcium-phase crystals of calcium oxalate and calcium phosphate, as well as promoting the removal of mineral precipitates via the urine. The composition of the invention comprising rustyback extract drastically reduces stone crystallisation by promoting the retention of oxalates in the dihydrate form, which generally do not cause stones but remain in solution in the urine of healthy subjects without generating pain and infection. The composition therefore also prevents stones. Indeed, stones contain small amounts of embedded proteins that act as adhesive bridges between crystals in mineral aggregates, and this protein component is of vital importance in the genesis of stones since the adhesion of crystals to cells depends on the bonds established with the membrane and the solubility of the crystal itself.

The composition of the invention comprises rustyback extract in an amount in the range of 50 to 150 mg, preferably about 100 mg, with respect to a unit preparation dose. The composition of the invention also includes a Protium heptaphyllum extract. Protium heptaphyllum is a plant belonging to the burseraceae family. It produces an amorphous resin whose main constituents are α and β amyrin. The composition of the invention comprises amyrines of Protium. The alpha and beta amyrines contained in protium extract have an anti-inflammatory and pain-relieving action that is useful in reducing the pain that accompanies the acute phases of stones. In particular, Protium reduces the release of inflammatory markers such as interleukins and TNF-α, which are responsible for inflammatory progression leading to the release of pain mediators. In conjunction with the other ingredients in the composition, it has demonstrated anti-inflammatory activity that is beneficial in the treatment of stones.

The composition of the invention comprises Protium Heptaphyllum extract in an amount in the range of 50 to 150 mg, preferably about 100 mg, with respect to a unit preparation dose.

The composition of the invention also comprises a dry origanum extract comprising 1% saponins.

The active ingredients mostly present in origanum extract are alkaloids, saponins, coumarins, terpenes, sterols, tannins and flavonoids. Of these, saponins in particular have an anti-crystallizing effect on calcium oxalates and phosphates. The composition, therefore, promotes the retention of oxalates in the form of calcium oxalate dihydrate (COD). This light-coloured and small-sized form of oxalate tends to form crystals that are more soluble in urine and are unlikely to melt and stratify, increasing in size with the risk of obstructing the urinary tracts. In contrast, the form of calcium oxalate monohydrate (COM), which is more common in acute stones, involves the presence of dark-coloured crystals that are much harder and less soluble in urine than COD.

The composition of the invention may thus comprise origanum extract in an amount of 25 to 50 mg, preferably about 25, with respect to a unit preparation dose.

The composition of the invention may also comprise further ingredients which improve its activity.

The composition of the invention also comprises in an advantageous embodiment a dry extract of horsetail comprising 7% silica.

Horsetail is rich in silicon, which accounts for about 10% of the plant's dry weight. There are two different types of silica in the plant: the organic-colloidal or soluble silica, which forms cytoplasmic colloids and is bound to proteins, lipids and starches, and the insoluble or mineral silica, which is deposited in epidermal concretions and within cell membranes. The human body contains approximately 8-10 g of silicon, an amount that tends to decrease with age; in particular, it is found in the thymus, adrenal glands, pancreas, spleen, lungs, bones, tooth enamel, skin and skin adnexa. The elimination of silicon is mainly renal and is related to that of calcium and magnesium, probably in the form of orthosilicate. The composition of the invention also includes a dry horsetail extract which has a diuretic action by stimulating the removal of fluids through the urine. This action is particularly useful in cases of urinary tract stones since, on the one hand, good hydration and proper removal of water through the urine reduce the precipitation of phosphate crystals and calcium oxalates, and, on the other hand, this action promotes the expulsion of any stones already formed that have arrived in the bladder and will be removed through the urine.

The composition of the invention therefore turned out to be a selected and synergistic combination of active ingredients capable of treating and preventing stones.

According to a primary aspect of the invention the invention concerns the use of the composition comprising the composition of the invention for use as a medicament.

According to a further aspect the invention concerns the composition described above for use in the treatment and prevention of stones.

The invention also relates to a supplement comprising the composition of the invention and excipients for use in the treatment and prevention of stones.

The composition and the supplement for use in the treatment and prevention of stones may preferably be administered by oral administration.

The compositions may then be in liquid, solid or semi-solid form and may be in the form of tablets, beads, drops, sticks, syrups, sachets.

The compositions of the invention are orally administrable in the form of tablets, capsules, granules, effervescent granules, syrups or the like.

They may also be dissolvable in water to form inhalable solutions.

The compositions in solid form may comprise one or more additives such as starches, sugars, cellulose, diluents, granulating agents, lubricants, binders, disintegrating agents, stabilizers, emulsifiers, texturizers, plasticizers, wetting agents, thickeners, coatings. All these excipients allow to obtain the supplement of the invention in the desired pharmaceutical formulation.

The capsules may be coated with a gastro-resistant film, or they may be soft gel capsules.

Flavouring agents, preservatives, dyes or similar agents may also be present. Preferably the composition of the invention is administered orally, more preferably in sachet form.

The daily dose of the finished product is preferably one or two sachets per day.

In a preferred and advantageous form of administration, one sachet comprises
Potassium citrate 1000 mg
Magnesium citrate 500 mg
Phyllanthus extract 150 mg
Bromelain 100 mg
Nettle extract 25 mg
Rustyback extract 50 mg
Protium heptaphyllum extract 50 mg
Origanum extract 25 mg

The preferred administration of two sachets/day for the treatment of stones therefore envisages double dosing:
Potassium citrate 2000 mg
Magnesium citrate 1000 mg
Phyllanthus extract 300 mg
Bromelain 200 mg
Nettle extract 50 mg
Rustyback extract 100 mg
Protium heptaphyllum extract 100 mg
Origanum extract 50 mg

The active substances contained in the compositions subject-matter of the present invention can be combined or mixed as active substances and/or excipients known according to the pharmaceutical and food or nutritional industry techniques and prepared by methods known in the pharmaceutical or food industry.

The compositions object of the invention are also used as adjuvants in the therapies for the treatment of stones.

In an advantageous aspect of the invention, the composition of the invention may be combined with at least one drug or surgical treatment. In fact, the invention can be combined with useful drugs for the management of stones such as allopurinol, diuretics, antispasmodics, antibiotics or NSAIDs. In addition, the invention can be combined with shock wave treatment for stones in order to reduce the size thereof prior to lithotripsy and improve patient compliance.

The composition of the invention can in fact be administered alone or in combination, i.e. as a co-therapeutic agent in a fixed-dose combination or in a combination therapy of separately formulated active ingredients, with at least one drug chosen from the group consisting of allopurinol, a diuretic, an antispasmodic, an antibiotic and a non-steroidal anti-inflammatory drug (NSAID).

In fact, the composition of the invention, i.e., the medicament or supplement comprising the same, exhibits excellent activity in the prevention and treatment of kidney stone formation, myorelaxation, regulation of uric acid precipitation, oxalate crystal scaling and anti-edema effect. Specifically, the product of the invention exhibited excellent inorganic breakdown activities of already precipitated oxalates, a characteristic that suggests its effective use in the treatment of acute stones. In addition, the invention exhibited excellent activity in reducing the precipitation of calcium oxalates in a supersaturated environment, which suggests its effective use in the prevention of stones.

In particular, the composition of the invention is therefore useful as a dietary and nutritional supplement and as a medicament for the prevention and treatment of stones.

The compositions according to the invention are therefore pharmaceutical, nutraceutical or food supplement preparations that can be introduced into the diet of an individual suffering from stones or presenting one or more of the symptoms of stones or can be used as a preventive measure in an individual with a family history of stones.

The composition according to the present invention is therefore a valid therapeutic tool for the management of the symptoms of stones.

In this sense, the composition of the invention also has a preventive action, as it prevents the precipitation of oxalates in the renal tubules and urinary tracts due to the presence of citrates. In addition, it promotes the retention of oxalates in their most soluble form in dihydrate urine.

The synergism generated by the combination further above has showed its efficacy in enhancing the effects of its components which resulted being greater than the sum of the known effects of the individual active substances.

### EXPERIMENTAL PART

### Example 1: preparation of the composition of the invention

For the preparation of the composition of the invention and therefore of the final product, such as for example a supplement, the following were employed:
- Potassium citrate 1000 mg
- Magnesium citrate 500 mg
- A dry extract of Phyllanthus with 15% tannins 150 mg
- Bromelain 100 mg
- A nettle extract 25 mg
- A rustyback extract 50 mg
- A Protium heptaphyllum extract 50 mg
- A dry extract of Origanum 1% saponins 25 mg

All quantities were expressed in relation to unit dose preparations.

The compositions of the invention could be formed by combining the above ingredients in the amounts indicated.

### Example 2 Evaluation of the composition of the invention.

The composition of Example 1 was tested in an *in vitro* and *in vivo* study for the evaluation of a nutraceutical formulation on kidney stones and their symptoms.

The study was carried out at the Department of Pharmacy, University of Naples "Federico II".

Evaluations were carried out to demonstrate the product's ability to be used in the treatment of stones.

The study was aimed at evaluating the ability of a nutraceutical formulation to prevent and treat the formation of kidney stones, myorelaxation, precipitation of uric acid, oxalate crystal scaling and anti-edema effect.

### Assay for the evaluation of effects on stone formation

### Materials and methods

To test for a possible stone-prevention effect, a control solution of 1 M CaCOs (50 mL) was prepared to which oxalic acid was added in increasing amounts until a precipitate in the form of crystals was obtained. Precipitation was achieved with 0.0116 g oxalic acid. To check for a possible treatment effect on stone formation, a control solution of 1 M CaCOa (50 mL) was prepared, to which 50 mg oxalic acid was added to obtain a precipitate at the bottom of the tube of h=1.2 cm after centrifugation.

### Results

### Simulation of prevention effect.

Potassium citrate, magnesium citrate, phyllanthus extract and rustyback extract were added to the 1 M CaCOs (50 mL) solution in the amounts of 200 mg, 100 mg, 30 mg, 10 mg, respectively. Then, oxalic acid was added in increasing amounts until a precipitate in the form of crystals was obtained. Precipitation was achieved with 0.0132 g oxalic acid. The result indicated the ability of the active ingredients to reduce oxalic acid crystal formation by -15.7% compared to the control. To the same 1 M CaCOs (50 mL) solution the entire composition of the invention shown in Example 1 was added, but in an amount equal to one-fifth, while maintaining all the proportions between the active ingredients, i.e:
- Potassium citrate 200 mg
- Magnesium citrate 100 mg
- A dry extract of Phyllanthus with 15% tannins 30 mg
- Bromelain 20 mg
- A nettle extract 5 mg
- A rustyback extract 10 mg
- A Protium heptaphyllum extract 10 mg
- A dry extract of Origanum 1% saponins 5 mg

Oxalic acid was then added in increasing amounts until a precipitate in the form of crystals was obtained. Precipitation was achieved with 0.0196 g oxalic acid. The result indicated the ability of the composition of the invention to reduce oxalic acid crystal formation by -72.8% compared to the control. The presence of all the ingredients therefore resulted in a clear synergism in the prevention of stones.

### Simulation of treatment effect.

Potassium citrate, magnesium citrate, phyllanthus extract and rustyback extract were added in the amounts of 200 mg, 100 mg, 30 mg, 10 mg, respectively, to the test tube containing 1 M CaCOs (50 mL) solution and a precipitated bottom of calcium oxalate crystals of h=1.2 cm. The mass was then left to rest for 24 h and then centrifuged. The precipitate at the bottom of the test tube measured h=0.9 cm. The result indicated the ability of the active ingredients to reduce oxalic acid crystal precipitate by -19.1% compared to the control. To the same test tube containing the 1 M CaCOs (50 mL) solution and a precipitated bottom of calcium oxalate crystals of h=1.2 cm, the entire composition of the invention shown in Example 1 was added, but in an amount equal to one-fifth, while maintaining all the proportions between the active ingredients, i.e:
- Potassium citrate 200 mg
- Magnesium citrate 100 mg
- A dry extract of Phyllanthus with 15% tannins 30 mg
- Bromelain 20 mg
- A nettle extract 5 mg
- A rustyback extract 10 mg
- A Protium heptaphyllum extract 10 mg
- A dry extract of Origanum 1% saponins 5 mg

The mass was left to rest for 24 h and then centrifuged. The precipitate at the bottom of the test tube measured h=0.6 cm. The result indicated the ability of the active ingredients to reduce oxalic acid crystal precipitate by -50.1% compared to the control.

### Assay for evaluation of anti-edema effect

### Materials and methods

Spontaneously hypertensive rats (SHR) (Charles River, Calco, Italy) of approximately 4 weeks of age were used. All the animals were kept in a controlled environment with constant temperature (24 ± 1 °C) and humidity (60 ± 5%), subjected to an artificial 12-hour day/night circadian clock with free access to food and water. The rats were treated according to the rules dictated by the Guide for the Safety and Use of Laboratory Animals (NIH publication 68-23 revised 1985) and the Local University Ethics Committee and Ministry of Health (authorisation no. 156/2017 -PR).

A number of 4 rats were assessed after one week of hospitalisation (at 5 weeks of age) for hypoperfusion-induced vascular damage and reperfusion injury under normotensive conditions.

The animals were anaesthetized by intraperitoneal injection of α-chloralose (50 mg/Kg p.c., i.p.) and maintained with additional injections (α-chloralose 30 mg/Kg p.c., i.v.). They were tracheotomized and mechanically ventilated. Subsequently, the renal arteries were isolated to induce hypoperfusion by clamping. Two catheters were inserted into the renal artery and vein respectively. The arterial catheter was used to take blood samples every 30 minutes to assess the concentration of gas in the blood. The venous catheter was used to administer additional anaesthesia and fluorescent tracers [dextran-bound fluorescein isothiocyanate, 70 kD MW, FITC dextran (FD 70), 50 mg/100 g p.c., i.v. as a 5% w/vol solution in 3 min, administered once at the start of the experiment 30 min after stabilisation of the preparation; rhodamine 6G, 1 mg/100 g p.c. in 0.3 mL, as a bolus with additional injection during BCCAO and reperfusion (final volume 0.3 mL100 g-1h-1)].

Observation of the circulation was performed using a fluorescence microscope (Leitz Orthoplan) equipped with long-distance objectives [2.5x, numerical aperture (NA) 0.08; 10x, NA 0.20; 20x, NA 0.25; 32x, NA 0.40, a 10x eyepiece] and a filter block (Ploemopak, Leitz); a 100 W mercury lamp provided illumination using filters for FITC, Rhodamine 6G and a thermal filter (LeitzKG1). Images of the vascular network were televized with a DAGE MTI 1000 low light camera and recorded by a computer-based frame grabber (Pinnacle DC 10 plus, Avid Technology, MA, USA). A computer-aided imaging software system was used.

Increased permeability and subsequent edema were calculated as normalized grey levels (NGL): NGL = (I - Ir)/Ir, where Ir is the average baseline grey level at the end of vessel filling with fluorescence (average of five windows located outside the blood vessels with the same windows used throughout the experimental procedure) and I is the same parameter at the end of hypoperfusion or at the end of reperfusion. The MIP Image programme (MIP Image, CNR, Institute of Clinical Physiology, Pisa, Italy) was used to determine grey levels ranging from 0 to 255 in five regions of interest (ROIs) measuring 50 × 50 mm (10× objective). In order to identify the same ROI position during the recordings along the vascular networks, we used a computer-assisted XY movement device of the microscope.

The active ingredients were administered as a suspension in the form of a gastric gavage mode, 10 minutes before the operation by occlusion and reperfusion.

### Results

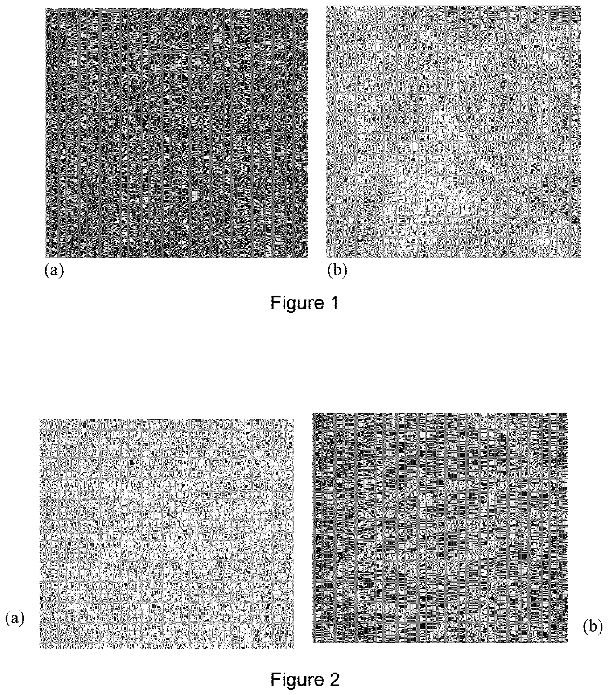

Figure 1 shows the increased vascular permeability and subsequent edema after ischemic damage induced by bilateral renal artery occlusion for 30 minutes and subsequent reperfusion, resulting in fluorescent dextran extravasation and vasoconstriction.

At the end of reperfusion, a marked increase in vascular permeability was evident (NGL= 1.23 ± 0.03, p < 0.01 compared to baseline conditions, NGL=0.11 ± 0.02).

Oral administration of bromelain (17.6 mg/kg) promoted a reduction in extravasation (NGL=0.91 ± 0.03, -26.1%), indicating a reduction in capillary permeability and thus an anti-edema effect (Fig. 2).

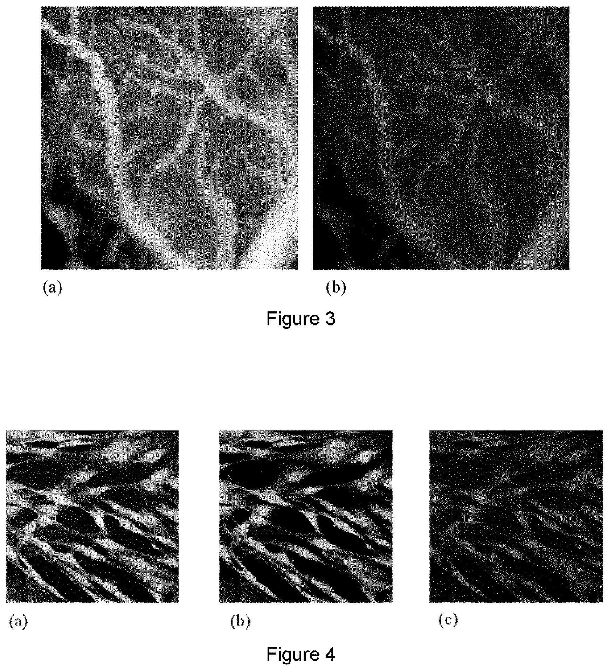

The oral administration of the entire composition potassium citrate, magnesium citrate, Phyllanthus extract, rustyback extract, Bromelain, Nettle extract, Protium extract, Origanum extract (amounts in mg respectively: 176.2, 88.1, 29.3, 38.1, 76.2, 4.4, 38.1, 4.4, out of a total of 454.8 mg/kg) promoted a more significant reduction in extravasation (NGL=0.40 ± 0.09, -67.1%), indicating a more intense reduction in capillary permeability and the edema phenomenon (Fig. 3).

### Assay for the evaluation of the muscle relaxant effect

### Materials and methods

To perform the assay, human pulmonary artery smooth muscle cells (HPASMC, Thermo Fisher Scientific, Monza, IT) were used. These are primary cell lines cryopreserved at the end of tertiary culture. The cells were cultured in smooth muscle cell medium (SMCM) (ScienCell, Carlsbad, CA, USA), in six-well plates pre-stratified with 0.1 mg/ mL poly-L-lysine (PLL) (Sigma-Aldrich) and placed in a humidified incubator with 5% CO2 air, at a temperature of 37 °C. After 48 h, the wells were gently washed with phosphate-buffered saline (PBS) and 2 mL of SMCM was added. The medium was replaced every 2-3 days. The cells were treated with 0.5% trypsin/EDTA and continuously cultured in SMCM.

The cells were identified by alpha smooth muscle actin (α-SMA) staining. The cells were fixed with 4% paraformaldehyde. After permeation with 0.3% (v/v) Triton X-100 (Sigma) for 5 min and sealing with 5% BSA-PBS for 1 h, the cells were incubated with rabbit anti-α SMA (Abcam, ab124964, Shanghai, China) 1:100 for 12 hours at 4°C; they were then washed three times with PBS. Goat anti-rabbit 549 (KPL, 072-04-15-06) 1:200 was used to visualize the binding of the primary antibody. Nuclear staining was performed with 4',6-diamidino-2-phenylindole dichlorhydrate (DAPI) (Sigma; 100 ng/ mL). Images were visualized using fluorescence microscopy (ECLIPSE TE2000-U, Nikon, Japan).

The cells were placed on a confocal plate (MatTek, Ashland, MA, USA) and incubated with Fluo-3/am (5 µM; Thermo Fisher Scientific) at room temperature for 30 min. After rinsing in buffer containing Ca2+ or free from Ca2+ for 20 min, the confocal plate was mounted on LSM 510 (ZEISS, Oberkochen, Germany) and the buffer was perfused during the experiment (1 mL/min, 23°C). The cells were stimulated with ACh (10-6 mM, TOKYO Chemical Industry, Tokyo, Japan) for 10 s and then sprayed with the respective buffer, as above (buffer containing Ca2+ or free from Ca2+). The ratio of fluorescence emission at 505 nm to excitation at 488 nm was measured. The parameter settings were Pinhole 544, detector gain 612, scan time 786.59 ms.

Fluorescence intensity measurements were recorded by selecting regions of interest as basic calcium fluorescence (BCF, mean value of stable fluorescence before stimulation with ACh), fluorescence difference (FD), post-peak basic calcium fluorescence (PPBCF, mean value of stable fluorescence after stimulation with ACh), peak time (PT) and total calcium activity time (TCAT). The formulae of FD, PT and TCAT were as follows. FD = Maximum fluorescence intensity: BCF. PT = Peak Fluorescence Time: Initial peak fluorescence time. TCAT = PPBCF time in fluorescence activity: BCF time in fluorescence activity. The buffer containing Ca2+ comprised the following (in mM): 160 NaCl, 2,5 KCI, 5 CaCl₂, 1 MgCl₂ 6H₂O, 10 HEPES and 8 glucose (adjusted to pH 7.3-7.4 with Tris-Base). Buffer free from Ca²⁺ comprising the following (in mM): 135 NaCl, 5 KCI, 1 MgSO4 7H2O, 5 HEPES, 10 Glucose and 1 EGTA (adjusted to pH 7.3-7.4 with Tris-Base).

### Results

Figure 4 shows the fluorescence of intracellular calcium ([Ca2+]i) in cultured smooth muscle cells. Stimulation of the cells with acetylcholine (ACh) promoted the entry of calcium into the cell, bringing the [Ca2+]i to a value of 51.5 ± 8.9 nM. Incubation with the mixture of nettle and origanum extract resulted in a significant decrease in fluorescence, corresponding to a decrease in [Ca2+]i to a value of 43.8 ± 7.2 nM (-15.0%). Incubation with the composition of the invention potassium citrate, magnesium citrate, Phyllanthus extract, rustyback extract, Bromelain, Nettle extract, Protium heptaphyllum extract, Origanum extract (in amounts of µg/mL: 200, 100, 30, 10, 20, 5, 10, 5, respectively) resulted in a further decrease in fluorescence, corresponding to a decrease in [Ca²⁺]i to a value of 25.7 ± 9.2 nM (-50.0%).

### Assay for assessing the effect on uric acid clearance

### Materials and methods

Human renal tubular epithelial HK2 cell lines were purchased from Nanjing Cobioer Biotechnology Co., Ltd. (Nanjing, China). HK2 cells were cultured on RPMI 1640 medium containing 10% FBS, penicillin (100 U/mL), and streptomycin (100 µg/mL) at 37 °C with 5% CO2. They were regularly subcultured with 0.25% trypsin-EDTA for 3-4 days.

Cell lysates were prepared and the protein concentration was quantified using the BCA assay kit (Beijing Dingguo Changsheng Biotechnology CO., Ltd. Beijing, China). Equal amounts of protein were separated with 10% sodium dodecyl sulphate (SDS)-polyacrylamide gel and then transferred onto PVDF membranes (Millipore CO., Ltd. Beijing, China). The membranes were blocked in Tris/Tween 20 buffered saline (TBS-T) containing 5% skimmed milk for 1 hour and incubated with primary antibodies against URAT1, OAT1 and β-actin overnight at 4 °C. After washing with TBS-T buffer, the membrane was incubated with the secondary antibody. Specific bands were visualized on gel imagers (Bio-rad, USA) by chemiluminescence using ECL^{™} detection reagents (Abbkine Biotechnology Co., Ltd., Wuhan, China).

### Results

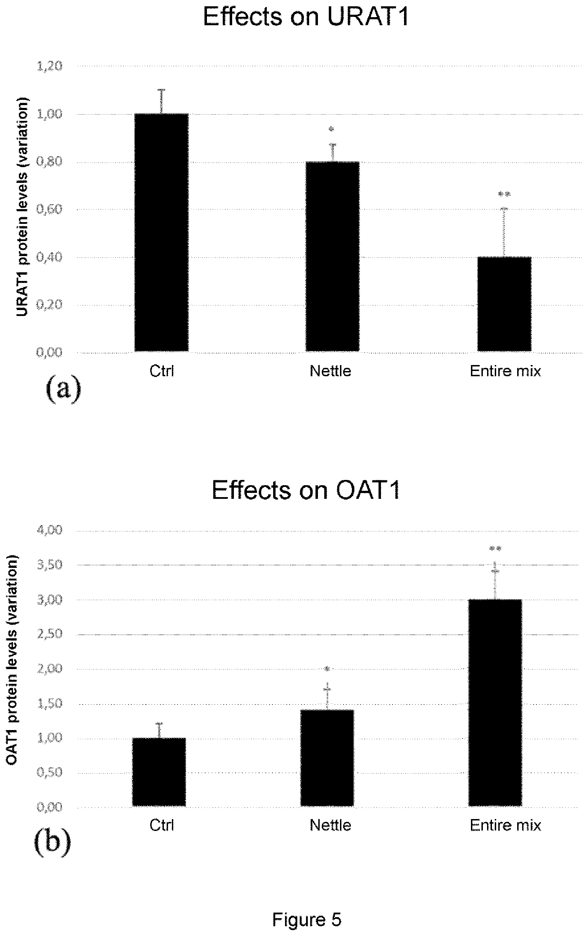

The URAT1 receptor reabsorbs urate in the lumen through the exchange with intracellular anions such as lactate and nicotinate, whereas the OAT1 receptor, expressed on the basolateral membrane, mediates the exchange of intracellular anions such as dicarboxylates. Figure 5 shows the results of the effects on URAT1 and OAT1 receptor expression following incubation of HK2 lines with the active ingredients. Nettle extract (5.0 µg/mL) alone promoted a decrease in URAT1 expression of around 20% (Fig. 5a) and an increase in the expression of OAT1 (Fig. 5b) by 40%, wherein the composition of the invention potassium citrate, magnesium citrate, Phyllanthus extract, rustyback extract, Bromelain, Nettle extract, Protium heptaphyllum extract, Origanum extract (in amounts of µg/mL: 200, 100, 30, 10, 20, 5, 10, 5, respectively) promoted a decrease in URAT1 expression of around 60% (Fig. 5a) and a 200% increase in OAT1 expression (Fig. 5b).

### Calcium oxalate crystal size evaluation assay

### Materials and methods

A sample of human urine was made in the laboratory with the following composition: water, 96%; urea, 20%; sodium chloride, 10%; nitrogen, 10%. Precipitation of calcium oxalate was induced by adding 40 µL of 0.1 M sodium oxalate per mL of urine (corresponding to 0.536 mg), every 30 min (0, 30, 60 and 90 min) under stirring at 37 °C, resulting in a final concentration of 2.2 mg/ mL of urine. Each urine sample was divided into two aliquots, one of which was used as a control (crystallisation without active ingredients) while in the other, calcium oxalate precipitation was induced in the presence of active ingredients, which were added to the sample 30 minutes before the crystallisation process. The calcium oxalate crystals were analysed using Easy Viewer IC Vision (Mettler Toledo).

### Results

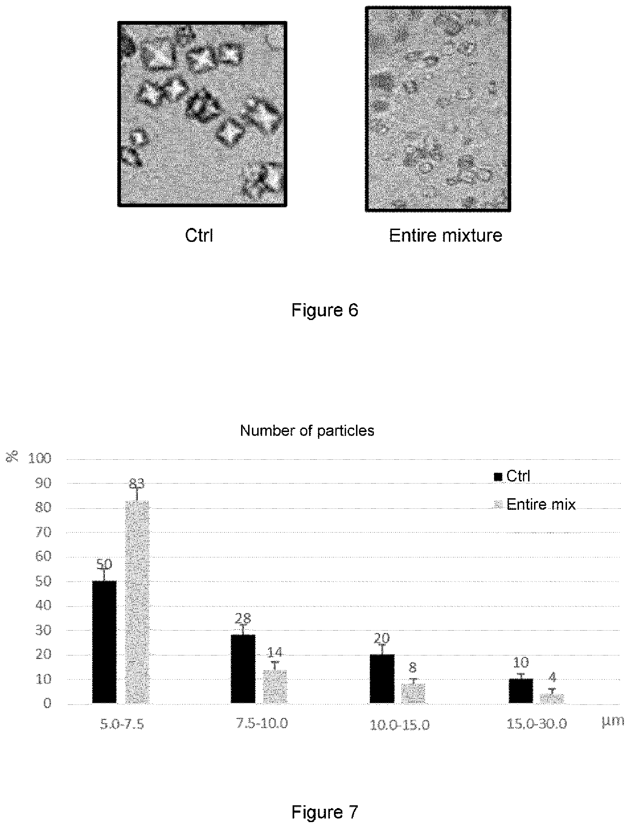

Figure 6 shows the size of the calcium oxalate crystals before and after addition of the composition, while Figure 7 shows the number of crystals (%) per size range (µm) in the various urine samples. It is clear that the composition of the invention potassium citrate, magnesium citrate, Phyllanthus extract, rustyback extract, Bromelain, Nettle extract, Protium heptaphyllum extract, Origanum extract (in amounts of mg/mL: 200, 100, 30, 10, 20, 5, 10, 5, respectively), is able to reduce the size of calcium oxalate crystals.

## Claims

1. A composition comprising potassium citrate, magnesium citrate, a phyllanthus extract, bromelain, a nettle extract, a rustyback extract, an origanum extract and a Protium heptaphyllum extract

2. The composition of claim 1, wherein the potassium citrate salt is present in the composition in the range of 1000 to 2500 mg, preferably about 2000 mg with respect to a unit preparation dose.

3. The composition of claim 1 or 2, wherein the magnesium citrate salt is present in the range from 500 to 1500 mg, preferably about 1000 mg, with respect to a unit preparation dose.

4. The composition according to any one of claims 1 to 3, wherein the phyllanthus extract, preferably comprising 15% tannins, is present in an amount of phyllantus in the range from 200 to 400 mg, preferably about 300 mg, with respect to a unit preparation dose.

5. The composition according to any one of claims 1 to 4, wherein bromelain is in an amount in the range of 100 to 250 mg, preferably about 200 mg with respect to a unit preparation dose.

6. The composition according to any one of claims 1 to 5, wherein the nettle extract is in an amount in the range from 25 to 100 mg, preferably about 50 mg, with respect to a unit preparation dose.

7. The composition according to any one of claims 1 to 6, wherein the rustyback extract is in an amount in the range from 50 to 150 mg, preferably about 100 mg, with respect to a unit preparation dose.

8. The composition according to any one of claims 1 to 7, wherein the Protium heptaphyllum extract is in an amount in the range of 50 to 150 mg, preferably about 100 mg, with respect to a unit preparation dose.

9. The composition according to any one of claims 1 to 8, wherein the origanum extract, preferably comprising 1% of saponins is in an amount of from 25 to 100, preferably about 50, with respect to a unit preparation dose.

10. A composition according to any one of claims 1 to 9 for use as a medicament.

11. A supplement comprising the composition according to any one of claims 1 to 9 and excipients.

12. A composition according to any one of claims 1 to 9 or a supplement of claim 11 for use in the treatment and prevention of urinary tract stones.

13. A composition according to any one of claims 1 to 9 or a supplement of claim 11 for use in the prevention and treatment of kidney stone formation, myorelaxation, uric acid precipitation, oxalate crystal scaling, anti-edema effect.

14. A composition according to any one of claims 1 to 9, wherein the composition is administered in combination, i.e. as a co-therapeutic agent, in a fixed dose combination or in a combination therapy of separately formulated active ingredients, with at least one drug selected from the group consisting of allopurinol, a diuretic, an antispasmodic, an antibiotic, and a nonsteroidal anti-inflammatory drug (NSAID).
